Europäisches Patentamt

European Patent Office

Office européen des brevets

(11) Publication number: **0 181 184**
A2

## (12) EUROPEAN PATENT APPLICATION

(21) Application number: **85307988.7**

(22) Date of filing: **04.11.85**

(51) Int. Cl.⁴: **A 61 M 35/00**
**A 61 J 1/00**

(30) Priority: **02.11.84 GB 8427770**

(43) Date of publication of application:
**14.05.86 Bulletin 86/20**

(84) Designated Contracting States:
**AT BE CH DE FR IT LI LU NL SE**

(71) Applicant: **Medipen Limited**
**10 East Parade**
**York YO3 7YL North Yorkshire(GB)**

(72) Inventor: **Brodrick, Andrew**
**10 East Parade**
**York YO3 7YL(GB)**

(74) Representative: **Behrens, Clemency Anne Susan**
**c/o CAS Behrens & Co Birstwith House**
**Birstwith Harrogate North Yorkshire HG3 2NG(GB)**

(54) **Drug dispenser.**

(57) A drug dispenser comprises a reservoir in which a drug substance is contained in solution. A tip or applicator is connected outside the reservoir and is in flow communication with an absorbent transfer member, which member sits in the reservoir and can absorb the drug solution and convey it to the tip. The arrangement is such that the drug solution is only able to leave the reservoir when contact is made between the tip and a solid surface, such as the body to be treated. On such contact a steady stream of drug solution can be applied to the solid surface by movement of the tip on the surface. The drug dispenser is designed to be used as a small hand-held unit.

EP 0 181 184 A2

Croydon Printing Company Ltd

-1-

The present invention relates to the application of a
drug solution and is particularly, but not exclusively
suitable for use in the medical and vetinary treatment
of the skin, mucous membranes, lips and related tissues.

In the past treatment of skin complaints has usually been
accomplished by the application of a drug preparation by
means of cotton wool, fingers or a paint brush to which the
solution or cream to be used is added by the user. None
of these is able to regulate easily the quantity of
preparation applied when the preparation is applied
in liquid form. Creams applied with the fingers carry the
additional problem of the risk of producing a secondary
infection or introducing dirt to the skin complaint that
is being treated, thus aggravating the situation.

According to the invention there is provided a single
unit comprising a reservoir containing a drug solution,
an applicator permanently connected thereto in normal
use, and means producing a steady release of the solution
through the applicator on contact with the tissues to be
treated.

The invention also provides a method of drug delivery to tissues by topical application of a drug substance in solution by contact with a suitable tipped container having a reservoir of the drug solution in close or direct association with the tip.

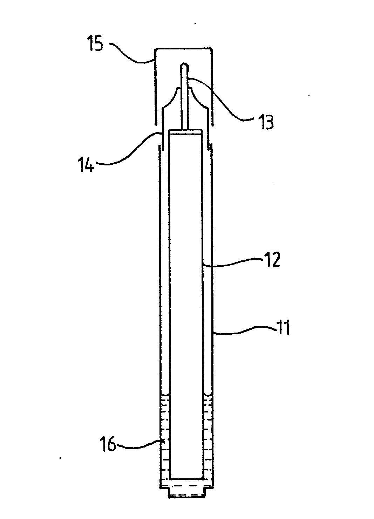

The invention will now be described, by way of example, with reference to the accompanying drawing, which is a cross section view of one embodiment of the invention.

The illustrated unit comprises a reservoir 11 of a suitable material, such as plastics material, to contain a drug substance in solution. The reservoir is preferably shaped as illustrated. A transorb unit 12, which is a supporting membrane through which the drug solution may pass to the site of application, is disposed within the reservoir 11 in contact with the drug solution. The transorb unit is preferably of cellulosic material.

A fibre tip, or other suitable tip, is mounted in the unit in direct contact with the transorb unit 12. The tip 13 provides for contact with the tissues being treated and acts as a carrier of the drug solution from the reservoir via the transorb unit 12. A closure unit 14 for the reservoir 11 fits snugly into the reservoir 11 to contain the drug solution and the transorb unit 12 and allows the tip 13 to protrude therethrough. A suitable capping device 15 encloses the exposed tip 13 when the unit is not in use to prevent loss of drug solution by evaporation or other methods from the exposed tip 13.

For use the unit containing the drug solution 16 is inverted to allow the solution 16 to pass to the tip 13. The tip is then put in contact with the tissues to be treated, which automatically leaves drug solution on the tissues.

The drug solution 16 will comprise a drug substance in solution with one or more of the following: water, alcohol, propyleneglycol, glycerol, acetone, pyrroldones. The solution 16 may also contain suitable preservatives, stabilisers and antioxidants.

-3-

as may be necessary to preserve the particular drug and maintain its stability.

The reservoir may be of any suitable size and material. However a preferred shape is a cylindrical vessel closed at one end and open at the other. An appropriate size could be a length of 90mm and a diameter of 15mm. The transorb unit 12 would then also be cylindrical and may fit into the reservoir with a gap of about 1-2 mm between its outer surface and the inner surface of the reservoir. Such a unit would contain about 4ml of drug solution. The tip 13 can be of any suitable size, but is preferably a solid cylindrical shape with a tapered leading edge. A suitable size is about 25mm by 2,5mm diameter.

It will be appreciated that this method can be used in any treatment from the application of antisceptic material to a cut or sore to the treatment of serious skin complaints. This is achieved simply and without mess with a single unit.

The drugs which may be usefully applied by this method are those which may be expected to have a use in dermatology, both human and animal. A list of such drugs includes:

A Germicides, Antibacterial agents and Antiviral compounds:
1. General. This category includes:
i) phenols (phenol, cresol, thymol, chlorxylenol,resorcinol pyrogallic acid, hexachlorophene)
ii) alcohols
iii) aldehydes (formaldehyde solution-formalin, hexamethylene tetramine
iv) acids (benzoic, boric, acetic)
v) halogens and halogenated compounds (iodine, iodophors, iodoform, povidone-iodine, hypochlorite, chlorhexidine, hydroquinolines, i.e. chinosol, halquinol chlorquinaldol, floraquin,diodoquin,chiniofon,clioquinol,chinoform,vioform)

vi) oxidising agents (hydrogen peroxide, zinc peroxide, potassium permanganate, benzoyl peroxide)

vii) metals and their compounds (organic mercurials, lead subacetate, selenium disulphide, silver sufadiazine, silver nitrate, zinc chloride, sulphate and oxide)

viii) surface active agents (anionic, nonionic, amphoteric and cationic; benzalkonium chloride, centrimede, domiphen bromide, dequalinium chloride)

ix) dyes (azo, acridine, fluorescein and triphenylmethane dyes, methylene blue)

10. Miscellaneous agents (sulfur, ichthammol, chrysarobin, anthralin, polynoxylin, cyclic salicylanilides).

2. Topical antibiotics

This category includes neomycin, framycetin, gramicidin, sodium fucidate, gentimycin sulfate, polymyxin B, and - particularly in the treatment of acne - tetracyline hydrochloride, erythromycin and clindamycin.

3. Topical antifungals

This group includes sodium propionate, undecylenic acid and its zinc salt, tolnaftate, salicylanilide, acrisorcin, benzoic and salicylic acids, gentian violet, potassium permanganate, phenolic compounds, imidazoles (miconazole, clotrimazole, thiabendazole), nystatin and candicidin.

4.                        parasiticides

~  Parasiticides (benzylbenzoate, gamma benzene hexachloride, mono-sulfiram, crotamiton, malathion).

5. Antiviral compounds

These include acyclovir, idoxuridine, amantadine and vidaralbine.

B. Anti-inflammatory agents

This category comprises the topical corticosteroids, a wide range of compounds which may be grouped into four categories:-

Group I     Extremely potent (e.g., clobetasol propionate 0.05%, halcinonide 0.1%).

Group II    Potent - most fluorinated preparations (e.g., betamethasone valerate 0.1%, fluocinolone acetonide 0.025%, triamcinolone acetonide 0.025%, 1%).

Group III   Moderately potent (e.g., clobetasol butyrate 0.05%, dexamethasone 0.01%).

Group IV   Less potent (e.g., hydrocortisone alcohol or acetate, 0.1-2.5%, methyl prednisolone 0.025%).

Nonsteroidal anti-inflammatories include bufexamac, benzydamine, ibuprofen, flurbiprofen and indomethacin.

C. Antihistamines

Examples include chlorcyclizine hydrochloride, diphenhydramine hydrochloride, mepyramine maleate and phenindamine tartrate.

D. Antipuritics and Local Anesthetics

Local anesthetics of the ester type include amethocaine, benzocaine and cocaine. with the amide type represented by cinchocaine, lignocaine and prilocaine. Other drugs with local anesthetic activity include benzyl alcohol, chlorbutol, camphor, menthol, phenol, pramoxine hydrochloride, dimethisoquin hydrochloride and some antihistamines.

E. Cytotoxic Agents

Physicians prescribe a number of topical cytotoxic agents to eradicate both superficial and malignant conditions of the skin as well as to treat benign, proliferative conditions. Drugs include 5-fluorouracil, podophyllin, colchicine, methotrexate, nitrogen mustard, nitrosourea and dinitrochlorobenzene.

F. Antiperspirants

Aluminium chloride and aluminium hydroxychoride form the mainstay of commercial antiperspirants: Formalin (formaldehyde solution), methenamine, glutaraldehyde and the anticholinergic agents homatropine methyl bromide and propantheline bromide have also been used.

G. Astringents

The main agents are tannins (tannic acid) and salts of aluminium (alum, acetate, acetotartrate, subacetate, chloride, sulfate) and zinc (chloride and sulfate).

H. Keratolytics and Caustics

Agents such as benzoic acid, salicylic acid, resorcinol and various thiols soften keratin and loosen cornified epithelium.

Alpha-hydroxy acids (citric, glycolic, lactic, malic, pyruvic and glucoronic), silver nitrate and sulfur act similarly.

I. Keratoplastic Agents

Keratoplastic agents soften and restore keratin: coal tar is used in atopic dermatitis, chronic eczema and psoriasis; vitamin A acid (tretinoin) and other retinoids are employed in acne as well as benzoyl peroxide; and protagonists for urea claim that it is a moisturizer and penetration enhancer; dithanol and its triacetate in psoriasis.

J. Rubefacients

These topical vasodilators are mainly the nicotinates (methyl, ethyl, butoxyethyl, phenethyl and thurfyl) and the essential oils (such as mustard, turpentine, cajuput and capsicum).

K. Pigmenting and Depigmenting Agents

The psoralens, applied topically or taken orally, are photoactive furocoumarins which increase the production of melanin on exposure to UV light. Dermatologists therefore use them to treat vitiligo. Trimethoxypsoralen and 8-methoxypsoralen are also used; the latter moreover is valuable in the treatment of psoriasis [PUVA (psoralen plus long-wave UV light) therapy] and mycosis fungoides.

Hydroquinonine and its monobenzyl ether interfere with the biosynthesis of melanin; they are used as depigmenting agents.

L.  Sunscreens

Sunscreens may be barriers (titanium dioxide, zinc oxide, red veterinary petrolatum) or screens (p-aminobenzoic acid, quinine, salicylate esters, cinnamates, anthranilates, mexenone and naphthol sulfonic acids).

The latter class is more suitable for incorporation into this invention

M.  Epilatories and Depilatories

Epilation refers to the complete removal of the hair from the follicle (plucking or wax treatment); depilation acts at skin level (strontium or barium sulfide, thioglycollic acid).

N.  Miscellaneous

There is a wide variety of emollients (oils, fats, hydrocarbons, waxes), demulcents (gums, mucilages, starches), protectives and absorbents, silicones and surfactants (anionic, cationic, nonionic, amphoteric).

2.  Penetration enhancers, accelerants and sorption promoters which could be included in the solution:

Such materials, if they are safe and nontoxic, may be used in the invention to enhance the penetration rate of drugs. Examples include dimethylsulphoxide, dimethylformamide, dimethylacetamide, alkyl sulphoxides, phosphine oxides, sugar esters, pyrrolidones (particularly 2-pyrrolidone, 1-methyl-2-pyrrolidone, 5-methyl-2-pyrrolidone, 1,5-dimethyl-2-pyrrolidone, 1-ethyl-2-pyrrolidone, 2-pyrrolidone-5-carboxylic acid and its salts), azocycloalkan-2-ones (particularly azone-1-dodecylazacycloheptan-2-one), tetrahydrofurfuryl alcohol, ethanol, propylene glycol (alone and in combination with other penetration enhancers), surfactants (anionic, cationic, nonionic and amphoteric), decylmethylsulphoxide, natural moisturizing factor (NMF), its analogues,

urea, dl-2-ethylhexylamine, and unsaturated long chain acids and alcohols such as oleic acid.

3. <u>Advantages of the       · . system</u>

The invention incorporates advantages for topical drug treatment such as economy of dosage, localisation of dose and application to diseased or damaged site only - all factors which minimize toxic side effects and promote correct drug usage as it would be difficult for the patient to overdose. The invention is readily portable, protects the contents from deleterious effects of air and light and is mechanically strong thus avoiding spillage which may occur with bottles, jars and tubes.

4. <u>Examples of suitable formulations for the invention</u>

The invention is widely applicable to many drug solutions. The examples quoted here refer to topical steroid formulations which have been tested by the in vivo vasoconstrictor assay in human volunteers and shown to be active.

    a)    Ethanol:water mixtures containing a suitable steroid - typical ethanol-water ratios lie between 50:50 and 70:30.

    b)    Propylene glycol:water mixtures containing a suitable steroid - typical propylene glycol-water ratios lie between 10:90 and 100:0.

    c)    Ethanol:propylene glycol:water mixtures containing a suitable steroid - typical formulations contained the following: ethanol, 56.5-13.0%; propylene glycol, 75.0-3.9%; and water, 27.0 -0%.

The steroid concentration may be typically between 0.001% and 1.0%. The following steroids were shown to be active in the formulation: betamethasone benzoate, betamethasone valerate, clobetasol propionate, triamcinolone acetonide, desonide, flumethasone pivalate, hydrocortisone butyrate and hydrocortisone. It is expected that other steroids would similarly be active in this type of formulation.

5. The following formulation has also been tested and found to be successful. The drug is gentian violet which is present as 1% weight /volume. The solvent is a mixture of ethanol (5%) and water. The ranges of drug and solution which are expected to be appropriate are:

gentian violet: 0.1-5%
ethanol: 1-10%
water: the balance.

If penetration enhancers are added to the solution this will reduce the weight/volume of the water required.

CLAIMS

1. A drug dispenser in the form of a hand-held unit comprising a reservoir containing a drug substance in solution, an applicator permanently connected thereto in normal use, and means producing a steady release of the solution through the applicator on contact with a solid surface.

2. A drug dispenser according to claim 1 wherein the means producing a steady release of solution comprises an absorbent transfer member disposed in the reservoir in contact with the solution and in flow communication with the applicator.

3. A drug dispenser according to claim 1 or 2 wherein the drug solution comprises a drug substance dissolved in a solvent, the solvent being one or more of the following:

water,alcohol,propylene glycol,glycerol, acetone, pyrrolidones.

4. A drug dispenser according to claim 3 wherein the solution contains an additive to promote penetration of the drug.

5. A drug dispenser according to any of the preceding claims wherein the drug substance comprises 0.0001% -5% of the weight by volume, and the solvent comprises water and alcohol.

6. A drug dispenser according to claim 5 wherein the alcohol content of the solution is 1-10% weight/volume.

7. A drug dispenser according to claim 6 wherein the drug substance is gentian violet.

8. A drug dispenser according to claim 3 wherein
the solvent comprises ethanol and water, the ethanol/
water ratios being between 50;50 and 70;30

9. A drug dispenser according to claim 3 wherein the
solvent comprises propylene glycol and water in the
ratios 1 ing between 10;90 and 100;0

10. A drug dispenser according to claim 3 wherein
the solvent comprises ethanol, propylene glycol and
and water, in the following ranges:
ethanol 56.5 -13.0% ; propylene glycol 75.0-3.9%;
and water 27.0-0%